# EUROPEAN PATENT SPECIFICATION

(11) **EP 1 292 703 B1**
(45) Date of publication and mention of the grant of the patent: **25.04.2007**
(21) Application number: 01902166.6
(22) Date of filing: 06.02.2001
(51) Int. Cl.: C12Q 1/68, C12Q 1/00, G01N 33/53, G01N 33/50, G01N 33/68

(54) **BIOMOLECULAR TOXICITY ASSAY**
BIOMOLEKULARER TOXIZITÄTSTEST
ANALYSE DE TOXICITE BIOMOLECULAIRE

(30) Priority: 07.02.2000 US 180826 P
(43) Date of publication of application: 19.03.2003
(73) Proprietor: Dos Remedios, Cristobal Guillermo, Paddington, New South Wales 2021 (AU); Kekic, Murat, Stanmore, New South Wales 2048 (AU); Cooke, Arthur Roger, San Francisco, CA 94117 (US)
(72) Inventor: Dos Remedios, Cristobal Guillermo, Paddington, New South Wales 2021 (AU); Kekic, Murat, Stanmore, New South Wales 2048 (AU); Cooke, Arthur Roger, San Francisco, CA 94117 (US)
(74) Representative: Jones, Elizabeth Louise
(86) International application number: PCT/AU2001/000099
(87) International publication number: WO 2001/059150

(56) References cited:
- WO-A-99/11814
- YONEZAWA N ET AL: "INHIBITION OF THE INTERACTIONS OF COFILIN DESTRIN AND DNASE I WITH ACTIN BY PHOSPHOINOSITIDES" JOURNAL OF BIOLOGICAL CHEMISTRY, AMERICAN SOCIETY OF BIOLOGICAL CHEMISTS, BALTIMORE, MD, US, vol. 265, no. 15, 25 May 1990 (1990-05-25), pages 8382-8386, XP002194596 ISSN: 0021-9258
- SMITH, D. R. ET AL: "High affinity renal lead-binding proteins in environmentally-exposed humans" CHEMICO-BIOLOGICAL INTERACTIONS , vol. 115, 1998, pages 39-52, XP001147452
- KEKIC MURAT ET AL: "Electrophoretic monitoring of pollutants: Effect of cations and organic compounds on protein interactions monitored by native gel electrophoresis." ELECTROPHORESIS, vol. 20, no. 10, July 1999 (1999-07), pages 2053-2058, XP009010339 ISSN: 0173-0835
- DATABASE CA [Online] MIKIO NISHIOKA; KUNIHIKO KOBAYASHI ET AL.: 'A binding activity of actin with human C1q', XP002962407 Database accession no. 97:196644 & BIOCHEM. BIOPHYS. RES. COMMUN. vol. 108, no. 3, 1982, pages 1307 - 1312, ISSN: 0006-291X
- E.W. DAOUD ET AL.: 'Inhibition of deoxyribonuclease I activity by actin covalently cross-linked to chick brain actin depolymerizing factor through exposed sulfhydryls' BIOCHEM. BIOPHYS. RES. COMMUN. vol. 155, no. 2, 1988, pages 890 - 894, ISSN: 10006-291X, XP002962436
- TOSHIHIKO TANAKA ET AL.: 'Calcium dependent regulation of the spectrin/actin interactio by calmodulin and protein 4.1' J. BIOL. CHEM. vol. 266, no. 2, 1991, pages 1134 - 1340, ISSN: 0021-9258, XP002962403
- MICHAEL C. SNABES ET AL.: 'A DNase I binding/immunoprecipitation assay for actin' J. BIOL. CHEM. vol. 256, no. 12, 1981, pages 6291 - 6295, XP002962404
- FRANZ GROLIG ET AL.: 'Partial characterization of a putative 110 kDa myosin from the green alga chara corallina by in vitro binding of fluorescent F-actin' CELL BIOL. INT. vol. 20, no. 5, 1996, pages 365 - 373, ISSN: 165-6995, XP002962405

## Description

### FIELD OF THE INVENTION

The present invention relates generally to an assay for the detection of toxicants. More particularly, the present invention contemplates an assay of toxicants such as those of the type comprising heavy metal, heavy metal divalent cations and organic molecules as well as organo-halides. Such toxicants are frequently present as contaminants in aquatic and terrestrial environments. The present invention further provides an assay device for detecting toxicants. The present invention is predicated in part on the sensitivity of binding partner affinity to the toxicants.

### BACKGROUND OF THE INVENTION

Bibliographic details of the publications referred to by author in this specification are collected at the end of the description.

A range of simple and complex chemical entities contaminate aquatic and terrestrial environments. Many of these molecules arise from the prolonged use of these or related or precursor molecules in a myriad of industrial processes. Such chemical entities, or toxicants, include heavy metal ions, organo-halides and organic molecules. The presence of toxicants can pose serious health risks to humans and animals and can also impact on vegetation and other participants in the biosphere. Some toxicants can also have a serious impact on the ozone layer. Accordingly, the reliable analysis of toxicants is important in monitoring the environment. It is also an important component of assessing the extent of contamination of industrial and urban sites which has implications for social, urban and industrial planning.

A range of assays have been developed for particular toxicants. Some of these assays require the use of complex and expensive apparatus and/or apparatus which is not portable or at least no readily portable. Furthermore, some assays are restricted in the range or type of toxicants which can be detected. There is a need, therefore, to develop further assays for toxicants which are sensitive and inexpensive.

One type of assay which has been developed is the bioassay. Bioassays can be classified into three categories: (i) whole metazoan animal assays such as assays of the rodents, fresh water copepods (crustacean) or the marine echinoderm assays (Nipper *et al*., 1997), or plant-based assays; (ii) single cell assays such as those based on light emitted from the luminescent marine bacterium, *Photobacterium phospherum,* commonly known as the Microtox test (Ruiz *et al*., 1997), and other single cell assays such as the lymphocyte assay (Markovic *et al.* 1977); (iii) subcellular assays using either beef heart whole mitochondria or sub-mitochondria particles in which membrane-based enzyme systems are used to assay the toxicity of samples (Read *et al*., 1997).

The range of tests currently approved by the Environmental Protection Agency (EPA) in the United States is limited to only the first of these categories. For example, the EPA has nominated only eight taxa for freshwater toxicity testing and a similar number for the testing of marine toxins.

There is a need, therefore, to develop an assay which can widen the analysis base for monitoring environmental pollution. For example, in the case of heavy metal ions, the EPA has set standards that described the total amount of metal ion in a sample. However, biological cellular systems separate their working biological molecules (cytoplasm) from the environment using a semi-permeable cell membrane (plasmalemma). It follows that this selectively permeable cell membrane may or may not allow a potentially polluting molecule or atom to pass through it and thus potentially toxic pollutants may or may not be toxic.

Furthermore, pollutants that are toxic in some biological systems may not be toxic in other systems. Frequently the biotoxicity of a pollutant depends on whether the pollutant is present in combination with other chemicals in the sample, for example, organic solvents may increase the toxicity of certain pollutants. Lipids may also affect the ability of a solute to be toxic (Pollak, 1998).

In work leading up to the present invention, the invention sought to develop an assay which could provide a rapid, reliable and a "yes/no" approach to determining whether a particular sample contained a toxicant or was likely to contain a toxicant. The assay developed by the inventors is a new type or class of assay, namely a molecular bioassay in which the formation of a complex between two macromolecules (naturally occurring and/or synthetic) forms the basis for sensing the presence of toxic ligands.

### SUMMARY OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

One aspect of the present invention provides a method for detecting an inhibitor of interaction between two or more binding partners said method comprising contacting the binding partners before, during or after said partners have formed a binding partnership with a sample putatively containing said inhibitor and screening for either dissociation of binding between said binding partners or inhibition of binding between said binding partners wherein dissociation or inhibition of binding is indicative of the presence of an inhibitor.

Another aspect of the present invention contemplates a method for detecting an inhibitor of interaction between two or more binding partners said method comprising immobilizing a binding partner to a solid support and contacting a sample putatively containing said inhibitor before, during or after a binding partner of said first binding partner labelled with a reporter molecule binds to said immobilized partner forming a binding partnership and screening for either dissociation of binding between binding partners or inhibition of binding between binding partners wherein dissociation or inhibition of binding is indicative of the presence of said inhibitor.

Yet another aspect of the present invention is directed to an assay device for detecting an inhibitor of interaction between members of a binding partnership comprising two or more binding partners said assay comprising a solid support comprising a binding partner immobilized thereto and a binding partner labelled with a reporter molecule to said immobilized binding partner.

Still yet another aspect of the present invention provides an assay device for detecting an inhibitor of interaction between members of a binding partnership comprising two or more binding partners said assay comprising a solid support comprising a binding partner immobilized thereto and a container adapted to contain a binding partner of said immobilized binding partner which is labelled or capable of being labelled with a reporter molecule.

A further aspect of the present invention contemplates the use of binding partners of a binding partnership in the manufacture of an assay for the detection of an inhibitor of interaction between said binding partners. The assay may be adapted for quantitative or quantitative deformulation of the inhibitor.

### BRIEF DESCRIPTION OF THE FIGURES

**Figure 1** is a photographic representation showing color patterns displayed by EtBr and/or DNA applied to the nylon membrane and illuminated by UV (top panel) or an incandescent lamp (lower panel). All chemicals applied at 5 µl volume. Additions are: (1) 10 mg/ml EtBr; (2) 0.25 mg/ml EtBr; (3) 1 mg/ml DNA; (4) DNA + 10 EtBr; (5) DNA + 0.25 EtBr at high sensitivity.

**Figure 2** is a graphical representation showing changes in emission spectrum of the DNA/EtBr complex upon addition of HgCl₂. Excitation - 520 nm.

### DETAILED DESCRIPTION OF THE PREFERRED EMBODIMENTS

The present invention is predicated in part on the use of macromolecular binding partners of a binding partnership in an assay to determine the presence of an inhibitor of the binding partnership.

In a first aspect, the invention provides a method as described in claim 1. Also described is a method for detecting an inhibitor of interaction between two or more binding partners said method comprising contacting the binding partners before, during or after said partners have formed a binding partnership with a sample putatively containing said inhibitor and screening for either dissociation of binding between said binding partners or inhibition of binding between said binding partners wherein dissociation or inhibition of binding is indicative of the presence of an inhibitor.

Reference herein to an "inhibitor" means a molecule or chemical entity which can reduce or prevent binding between binding partners or which is capable of completely or partially dissociating binding partners after interaction. An inhibitor is preferably in the form of a toxicant. The term "toxicant" is used in its broadest sense to include any inhibitor of binding between binding partners or a molecule capable of promoting complete or partial dissociation of binding partners which have bound together in a binding partnership. Toxicants may or may not necessarily be "toxic" in the sense that they are capable of inducing death of a living organism or inducing one or more mutations in the genome of an organism or impairing physiological processes of an organism. Toxicants may also vary their toxicity depending on concentration or time of exposure. Preferred inhibitors in the form of toxicants include heavy metals, heavy metal ions and simple and complex organic molecules and organo-halides (2,4-dioxin or Picloram).

The sample to be tested may be a laboratory test sample, an environmental sample such as from an aquatic or terrestrial environment or may be an industrial sample from, for example, a site at or around an industrial process. A "terrestrial" sample includes gaseous samples such as air including ozone.

The assay of the present invention may be carried out in any number of ways, In one particularly useful embodiment, one member of a binding partnership is immobilized to a solid support. Another member of the binding partnership is then labelled with a reporter molecule. The binding partners are then allowed to bind. A sample putatively containing an inhibitor is then contacted with the immobilized binding partner-labelled binding partner complex. Loss of the reporter molecule is then indicative that the inhibitor has induced complete or partial dissociation of the binding partnership.

In another useful embodiment, the sample containing the putative inhibitor is brought into contact with the immobilized members of the binding partnership and labelled member of the binding partnership prior to binding of the partnership. Absence of the reporter molecule is indicative that the inhibitor is preventing or reducing formation of the binding partnership.

Generally, the binding partners represent pairs of molecules. However, the present invention extends to a binding partnership comprising three or more members.

The binding partners used in the method of the invention are a nucleic acid molecule and a dye. Alternative methods use macromolecules such as proteins. Reference herein to a "protein" includes reference to a peptide or polypeptide. A protein also includes an enzyme and molecules having protein components such as glycoproteins, fusion proteins and lipoproteins. With respect to enzymes, the binding partners may include the enzymes and an enzyme substrate. Accordingly, a binding partner may also be a non-proteinaceous molecule such as a substrate. The macromolecules may also be nucleic acid molecules including RNA or DNA or hybrid forms thereof. Reference to "RNA" and "DNA" includes reference to antisense and sense molecules and oligonucleotides as well as large nucleic acid molecules, A binding partnership may also comprise nucleic acid molecules and nucleic acid binding proteins.

The binding partners may alternatively comprise actin and actin-binding proteins such as but not limited to cofilin and DNaseI.

As stated above, in one preferred embodiment, one member of the binding partnership is immobilized to a solid support.

According to this preferred aspect of the present invention, there is provided a method for detecting an inhibitor of interaction between two or more binding partners said method comprising immobilizing a binding partner to a solid support and contacting a sample putatively containing said inhibitor before, during or after a binding partner of said first binding partner labelled with a reporter molecule binds to said immobilized partner forming a binding partnership and screening for either dissociation of binding between binding partners or inhibition of binding between binding partners wherein dissociation or inhibition of binding is indicative of the presence of said inhibitor.

Generally, the binding partner is immobilized to a solid support comprising glass or a polymer such as polystyrene, polymethacrylate, cellulose, polyacrylamide, nylon, polyvinyl chloride or polypropylene. The solid support may be in the form of tubes, beads, discs of microplates, or any other surface suitable for conducting an immunoassay. The solid support may be also considered as a solid matrix such as a wet or hydrated solid matrix. The binding processes are well-known in the art and covalent or passive binding may be employed.

By "reporter molecule" as used in the present specification, is meant a molecule which, by its chemical nature, provides an identifiable signal which allows the detection of binding between binding partners or dissociation of binding. The most commonly used reporter molecules in this type of assay are either enzymes, fluorophores or radionuclide containing molecules (i.e. radioisotopes) and chemiluminescent molecules.

Also described is an assay in which the binding partners are actin and an actin-binding protein such as cofilin or DNaseI. Globular or monomeric actin is immobilized to a solid support such as in the form of a test strip. Polymethacrylate or polystyrene are particularly preferred in this respect.

In practice, avidin or similar compound such as streptavidin is covalently fixed to the solid support. Actin is reacted with biotin to form biotinylated actin. The latter is then allowed to interact with the avidin/streptavidin-solid support so as to form a solid support comprising avidin/strepavidin-biotinylated actin. An actin binding partner such as cofilin or DNaseI is then labelled with a visible dye such as Texas Red (Molecular Probes, Inc.). The labelled actin binding protein is then allowed to bind to the immobilized actin and the test strip contacted with a test sample containing, for example, a toxicant such as one or more heavy metal ions (e.g. Cd, Cu, Hg or Zn). Dissociation of the coloured binding partner is then observed when a toxicant inhibits the binding partnership. Alternatively, visualization may be following electrophoresis such as described by Kekic and dos Remedios (1999).

As stated above, the assay may be varied in any number of ways. For example, a solid support may comprise a wet or hydrated solid matrix where the solid support retains one binding partner that binds to a second binding partner labeled with a coloured or fluorescent ligand dye. In another alternative, the assay involves detection of a visible reaction product. In this embodiment, the toxicant alters enzymatic activity such as in whole or fragmented mitochondria. Enzyme activity is then measured, for example, spectrophotometrically to monitor absorbance changes at specific wavelengths.

Also described is an assay based on the high affinity of toxicants such as cadmium and mercury for sulfhydryl groups which are present, for example, in many proteins. The binding to or reactive of heavy metals with sulfhydryl side chains of proteins has an effect on their reactivity.

In designing the assay method, it is particularly useful to select naturally-occurring biological molecules such as proteins and nucleotides. However recombinant or synthetic as well as derivative forms of these molecules may also be employed. Preferably, the binding partners interact with high affinity but be capable of interference by a range of toxins such as toxic pollutants. The term "interference" means that the interaction is altered in some significant way such as a decrease in binding affinity between the binding partners. Reference to "binding" such as in a binding partnership is intended to be broadly construed and includes covalent binding including disulphide linkages, ionic bonding, hydrogen bonding and bonding or association via van der Waal's forces and/or electrostatic attraction.

The interaction between the binding partners may be detected or monitored by any means including macroscopic and microscopic visualization. Macroscopic visualization includes visualizing light emissions and dyes as well as observing interaction between movable solid supports such as beads.

Also described is an assay device for detecting an inhibitor of interaction between members of a binding partnership comprising two or more binding partners said assay comprising a solid support comprising a binding partner immobilized thereto and a binding partner labelled with a reporter molecule to said immobilized binding partner.

An assay device for detecting an inhibitor of interaction between members of a binding partnership comprising two or more binding partners said assay comprising a solid support comprising a binding partner immobilized thereto and a container adapted to contain a binding partner of said immobilized binding partner which is labelled or capable of being labelled with a reporter molecule is also described.

The assay device may alternatively comprise containers adapted to contain agents useful in conducting the assay such as reporter molecules and diluents. A container adapted to contain a sample such as an environmental sample may also be included. The assay device may be packaged separately or all together and may further comprise means to detect the presence or absence of a reporter molecule. The assay device may also contain instructions for use.

A further embodiment of the present invention contemplates the use of binding partners of a binding partnership in the manufacture of an assay for the detection of an inhibitor of interaction between said binding partners. The assay may be adapted for quantitative or qualitative determination of the inhibitor.

The term "assay" may be read synomonously with "method'. Aspects of this invention relating to electrophoresis are encompassed by the description in Kekic and dos Remedios (1999).

The present invention is further described by the following non-limiting Examples in which Examples 1-4, 6-8, 10-11 and 14 are presented as illustrations of alternative assays and Examples 5, 9, 12 and 13 are illustrative of the invention.

### EXAMPLE 1

### Materials and Methods

Actin preparation, preparation of cofilin and DNaseI, native gel electrophoresis and prepared or heavy metal ions and organic compounds and other methods are as described in Kekic and dos Remedios (1999).

### EXAMPLE 2

### Inhibition of Interactions of Macromolecules

The inhibition of the interaction of, for example, actin and cofilin or actin and DNaseI can be visualized by immobilizing one macromolecule (for example, actin) to a solid support strip suspended in a small (1-2 ml) container. The sample is taken up into a syringe which serves two purposes: (1) to measure the volume of the potentially polluted water sample; and (2) the syringe contains a buffer compound which stabilized the pH of the aqueous sample. A small filter is then placed on the outflow of the syringe and the sample is injected into the vial containing the macromolecules. Dissociation of a coloured macromolecule (for example, a Texas Red (Molecular Probes Inc.)) dye attached to cofilin (or DNaseI or another actin-binding protein) can be visually observed to dissolve of the strip containing the non-coloured actin.

The experimental basis of this assay is described below.

Four heavy metal ions (Cd, Cu, Hg and Zn) were examined over the concentration range 1-1000 µg L⁻¹. The complex is most sensitive to progressive addition of 1-20 µg L⁻¹ Hg (added as the acetate). The volume densities of the actin-cofilin bands recorded from the native polyacrylamide gels are listed in Table 1. Addition of 1 and 5 µg L⁻¹ result in a progressive increase in the density of the actin-cofilin complex, whereas 10 µg L⁻¹ reduces the density of this band to levels comparable with the control with the simultaneous appearance of a series of slower bands. At 20 µg L⁻¹ this trend is accentuated with the almost complete loss of the native actin-cofilin band. The absence of unbound cofilin near the top of the gel suggests that native cofilin is not dissociated. From this and other determinations. The inventors estimate that the E₅₀ for Hg for the disappearance of the actin-cofilin band is approximately 20-40 µg L⁻¹ using 25 µM actin-cofilin complex. This binding ratio suggests that one Hg²⁺ binds to a highly-reactive cysteinyl residue in the actin-cofilin complex.

Cu²⁺ does not noticeably affect the actin-cofilin complex until it reaches a concentration of approximately 200 µg L⁻¹. At higher Cu²⁺ concentrations formation of the actin-cofilin complex is progressively inhibited and the proteins seem to disperse evenly along the gel lane. The 50% effective dose (E₅₀) for Cu²⁺ is approximately 400-600 µg L⁻¹. Cu²⁺ can bind to a number of amino acid side chains, for example, it is known to bind to Phe-375 of the actin sequence. Judging by the E₅₀ values, disruption of actin-cofilin complex is about an order of magnitude more sensitive to Hg²⁺ than to Cu²⁺.

The inventors tested the effects of adding ZnCl₂ using the concentration in the range 250-2500 µg L⁻¹. At these relatively high concentrations, it decreased the density of the actin-cofilin complex. The E₅₀ value (900-1000 µg L⁻¹) was significantly higher than either Hg or Cu. Thus, increasing sensitivity of the actin-cofilin band to these transition metal ions was achieved in the order: Hg>>Cu>Cd Zn.

**TABLE 1¹**

| Metal Cation | Actin-Cofilin µg L⁻¹ | SMP µg L⁻¹ | BHM µg L⁻¹ | Fish µg L⁻¹ (at 96 hr) | Microtox µg L⁻¹ |
|---|---|---|---|---|---|
| Hg²⁺ | 20-40 | 130 | 126 | 170 | 59 |
| Cu²⁺ | 400-600 | 300 | 93 | 530 | 9300 |
| Cd²⁺ | 800-1200 | 520 | 158 | 630 | 41400 |
| Zn²⁺ | 900-1000 | 1700 | 80 | 2990 | 33000 |

| | | | | | |
|---|---|---|---|---|---|
| ¹ Effective (E₅₀) concentrations for four heavy metal cations determined by: (1) Analysis of native PAGE gels of actin-cofilin; (2) Sub-mitochondrial Particle (SMP) assay; (3) Beef Heart Mitochondria (BHM) assay; (4) Whole Organism (Fish) assay; and (5) mean lethal concentrations (@ 96 hr) for *Photobacterium phosphoreum,* known as the Microtox assay. The amounts of added metal ions (g L⁻¹) did not include the mass of the counter ions (chlorides or acetate). Data for the right hand four columns were cited from Read *et al.* (1997). | | | | | |

### EXAMPLE 3

### Detection of Interaction

The actin-cofilin interaction can be monitored using a fluorescence-based detector system in which a donor fluorescent probe is excited by a light of the appropriate wavelength (using a suitable selective filter). In this example an Alexa (trade mark) fluorescent dye (used here as the donor probe) can be placed on one molecular (for example, monomeric actin) which in this case need not be fixed to a solid support. A second, different label (the acceptor probe) is reactivated with a second macromolecule (for example, cofilin), where the absorption spectrum of the acceptor is chosen to so that it overlaps the emission spectrum of the donor probe. Where there is close physical association between the actin and the cofilin an actin-cofilin complex, the emission of the donor probe is quenched by the acceptor probe and little or no fluorescence emission will occur. When the two proteins are physically dissociated (for example, a polluting toxicant), the donor emission is no longer quenched and fluorescence emission of the donor probe is detected by a photon detector. Thus, in this form of the device, the interaction between macromolecule ligands (for example, actin and cofilin) is monitored using fluorescence resonance energy transfer and the toxicant which dissociates the binding of these macromolecules is detected by the increase in light emission from the donor probe due to the removal of the acceptor probe.

### EXAMPLE 4

### Use of Magnetic Beads

Another form of the device relies on magnetic beads to separate the bound from the unbound proteins. Dynabeads (trade mark) provide a mechanism for sensing the binding between actin and cofilin. In this form of the device, actin is reacted with the magnetic beads and cofilin (labeled with Texas Red as above) is allowed to bind. The beads, therefore, hold actin and through actin they hold the cofilin. The proteins can be stored in this state either freeze dried or in a semi-dry state. This device works, by adding the aqueous sample to a small container containing a biological buffer. The container is fitted with a ring magnet which slides over the outside of the container and draws the magnetic beads to one end of the tube.

A water sample which failed to dissociate the coloured cofilin from the uncoloured actin is deemed safe or non-toxic. A sample which dissociated the two proteins is considered potentially toxic.

### EXAMPLE 5

### Nucleic Acid Sensing System

In this assay, a coloured indicator dye (e.g. acridine orange) is intercalated into the DNA or bound to a single-stranded nucleic acid such as RNA. Toxicity is indicated by the loss of the dye from an immobilized nucleic acid.

### EXAMPLE 6

### Electrophoresis of complexes of actin-cofilin or actin-DNaseI in native gels

Combinations of actin, cofilin and DNaseI were mixed at equimolar concentrations (~20-25 µm) in a Eppendorf tube and incubated for 15 min at 22°C. Actin, cofilin, DNaseI and their complexes were then separated on a 10% w/v native gel. Since the separations in this gel were achieved under nondenaturing conditions, the proteins do not migrate according to their apparent molecular weights as they do in SDS-PAGE gels. Instead, the rate of migration is determined by the ratio of charge to volume. Actin (*M*ᵣ 43 kDa) has a net negative charge. Under conditions of low ionic strength, monomeric actin is seen as a major monomer band migrating ahead of lesser amounts of dimer and higher oligomers up to the interface between the stacking (5%) and running (10%) gels. Cofilin is about half the molecular mass (18.7 kDa) of actin, but because of its net positive charge at the pH conditions in the native running gel (pH 8.6), it remains in the stacking gel. DNaseI (*M*ᵣ 43 kDa) migrates as an indistinct band moving ahead of the actin monomers. When cofilin and DNaseI are mixed in equimolar amounts, there is no evidence that the two form a complex.

An equimolar complex of actin with either DNaseI or cofilin produces two bands which are more sharply defined than any of the proteins alone. A mixture of actin with DNaseI results in two sharp bands, a lower band which migrates near the actin monomer and free DNase bands (but is not identical to either), and a slightly less dense upper band. A similar, but nonidentical pair of bands, is seen when actin is complexed to cofilin. Note the reduction in densities of the unbound DNaseI and cofilin bands. These actin-ABP complexes are the focus of electrophoretic forms of the assays described in Kekic and dos Remedios (1999).

### EXAMPLE 7

### Pollution test based on a protein-protein interaction where one of the proteins is immobilized on a solid matrix

In this Example, a pollution-sensitive device is described which is constructed using a solid support consisting of a wet or hydrated solid matrix where the solid support retains one protein that binds to second protein that is labeled with a colored (and or fluorescent) ligand dye and where the pollutant or toxicant inhibits the interaction of the two proteins.

The interaction of proteins like actin and cofilin has been shown to be sensitive to a number of common environmental pollutants (Kekic and dos Remedios, 1995). This Example describes a color reaction based on actin and cofilin that is easily adapted to field use and is sensitive to pollutants. The Example may also employ another actin-binding protein besides cofilin, for example, DNase I.

Cofilin is expressed as a fusion protein with a glutathione s-transferase (GST) tag. This tag allows cofilin to be bound very tightly to a matrix such as glutathione agarose which is placed in a transparent column. Actin is labeled with a highly visible fluorescent dye such as Texas Red and passed through this column. A number of these fluorescently labeled actin molecules will remain bound to the cofilin on the column and a bright reddish color will remain that is also easily visible by illumination with ultra violet light. This coloration remains on the column after passing purified low salt buffer through it. However, the color has been shown to disappear after a solution contaminated with mercury is passed through. The removal of color is almost certainly due to the mercury ions disrupting the interaction between the actin and cofilin. Hence, the "colored" actin is eluted from the column leaving behind the uncolored cofilin-agarose.

In practice, a polluted water sample could be detected either by the loss of coloration due to the release of a colored (labeled) second protein from the matrix or the presence of color in the eluent (or both).

### EXAMPLE 8

### Pollution test based on protein activity producing a visible reaction product

An established bioassay of polluted water samples is that based on altered enzymatic activity of either whole or fragmented mitochondria. Toxicants such as heavy metal ions cause a quantitative alteration in the enzyme activities contained in these mitochondria or mitochondrial fragments (so-called sub-mitochondrial particles). These enzymes are usually assayed in a spectrophotometer by monitoring absorbance changes at specific wavelengths. One of more of these mitochondrial enzymes is a suitable target for an enzyme-based assay in which activity is detected by the development of a visible, colorimetric reaction product.

### EXAMPLE 9

### Pollution test based on DNA-dye interaction

A similar device is constructed where the macromolecule is a nucleic acid like DNA bound to a solid support (for example, but not limited to treated or untreated glass beads) retained in a transparent tube, and where the binding partner is a dye, for example, but not limited to fluorescent labels such as acridine orange, ethidium bromide or fluorescein-based dye. Detection of a polluted water sample can be deduced by either the loss of color from the column or by detection of the label in the column eluent.

A pollution-sensitive device is constructed, therefore, using a solid support consisting of a wet or hydrated solid matrix where the solid support retains a nucleic acid polymer such as DNA or a stabilized RNA that binds to second colored (and or fluorescent) ligand dye and where the pollutant or toxicant inhibits the binding of the dye to the nucleic acid.

### EXAMPLE 10

### Test based on the reactivity of sulfhydryls

Heavy metals such as cadmium and mercury have a high affinity for sulfhydryl groups such as those found on many proteins. The binding to or reaction of heavy metals with sulfhydryl side chains of proteins has an effect on their reactivity. There are a number of established protocols for measuring the reactivity of sulfhydryls. One involves Ellmans reagent and another method involves the use of NBD-Cl (a fluorescent dye) (Akinyele *et al*., 1999. Another method involves the use of the fluorescent dye NBD-F. This dye is reported to have a higher fluorescent yield (likely to be more visible) and a greater reactivity (Watanabe and Imai, 1981).

Ligands may be used with any number of sulfhydryls (cysteine residues) or a cysteine solution to detect the presence of pollutants (such as heavy metals) by measuring the effect on the interaction between the fluorescent dye and the sulfhydryl. The color of the testing solution fades visibly depending on the magnitude of the effect between the pollutant and the sulfhydryl. This provides a field test giving a "yes/no" answer or, by using a detection device (e.g. light transmission or absorbance) a reading is obtained - giving a high or low value for the pollutant.

A pollution-sensitive device is, therefore, constructed using a solid support consisting of a wet or hydrated solid matrix where the solid support retains a protein with multiple cysteinyl side chains where the reactivity of these cysteinyls is reduced with pollutants or toxicants (for example, heavy metal ions).

### EXAMPLE 11

### Test based on the protein-protein interactions using a polyacrylamide gel-based assay using complex mixtures

Heavy metals and other xenobiotics (i.e. chemicals that are foreign to life) or toxicants are often non-toxic or minimally toxic when applied separately and individually to a biotoxicity assay but can have an unexpectedly large toxicity when applied in combination (Pollak, J.K. 1998).

An electrophoretic method is used to demonstrate differential toxicity in samples of water known to contain a range of pollutants.

The toxicity of combinations of chemicals (chemical mixtures) is detected by protein-protein (actin-cofilin) interactions monitored by native gel polyacrylamide gel electrophoresis or by other solution-state of solid state assay.

### EXAMPLE 12

### Immobilized DNA containing a dye/ligand is used to detect heavy metal ions

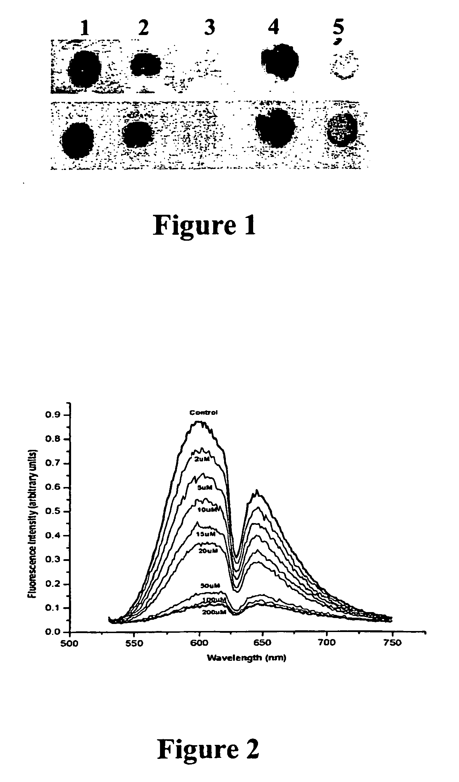

DNA is immobilized on a solid support such as but not limited to a membrane. An example of this membrane is a commercial nylon membrane product called Immobilon-Ny+ Transfer Membrane (purchased from Millipore Pty Ltd). DNA is capable of binding a number of fluorescent dyes such as acridine orange, ethidium bromide but the dyes alone do not bind. Sub-picogram quantities of DNA can be detected using this membrane. Binding of a dye such as ethidium bromide (EtBr) can be detected by simple visual inspection using ordinary daylight or an artificial incandescent lamp. Binding of a dye to DNA is also detected by illuminating the DNA with ultraviolet or near-ultraviolet light and the fluorescence visually observed (see Figure 1).

Toxicity of heavy metal ions, therefore, is detected by the inhibition of DNA-ethidium bromide interaction where the DNA is immobilized on a solid membrane support.

### EXAMPLE 13

### Heavy metal ions both inhibit and alter the emission maximum of fluorescence of DNA-ligand dyes

The fluorescence emission of DNA-dye ligands such as ethidium bromide (EtBr) are examined spectroscopically in solution.

5 µg/ml DNA and 2 µg/ml EtBr at same volume of 10 µl and emission spectra are taken as presented in Figure 2. Addition of HgCl₂ resulted in concentration-dependent quenching of fluorescent signal at range 1-100 µM. Changes in fluorescence intensity were noticeable at 2 µM.

The toxicity of heavy metal ions is detected, therefore, by the inhibition of DNA-ethidium bromide fluorescence where a significant decrease in fluorescence intensity can be readily noted using concentrations of heavy metal ions as low as 2 µM.

### EXAMPLE 14

### Heavy metal ions and/or other pollutants alter conformation of proteins and thus alter the spectral properties of an attached fluorescent dye

When the micro-environment surrounding a fluorescent dye attached to a protein (such as actin) is altered, this results in a change to the spectral properties of this dye. These changes are either visible with the naked eye or spectrophotometrically.

An example of this is cadmium-mediated depolymerization of pyrene-labelled actin filaments (Wang and Templeton, 1996). This is not necessarily limited to polymeric proteins and applies to the monomeric proteins (including actin) labeled with a fluorescent dye. Such an example is the reduction in fluorescence intensity of alpha-chymotrypsinogen A labeled with 1-anilinonaphthalene-8-sulfonic acid (ANS) when exposed to organic compounds such as alcohol (Khan *et. al*., 2000).

The presence of heavy metals, organic solvents and/or other pollutants is detected, therefore, from spectral changes in proteins (or other ligands) labeled with fluorescent dyes in response to conformational and/or structural changes to the protein or ligand.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

Akinyele *et al.,* 1999, *J. Agric. Food Chem 47:* 2303-2307
Kekic and dos Remedios, 1999, *Electrophoresis 20:* 2053-2058
Khan *et al*., 2000, *Biochim. Biophys. Acta 1481(2):* 229-236
Markovic *et al*. 1997, *Immunol. Cell Biol. 71* Supplement S3
Nipper *et al.* 1997, *Ecotoxicol 3:* 109-115
Pollak *et al.* 1998, *Int. J. Environm. Health Res. 8:* 157-163
Read *et al*. 1997, *Environmental Applications with Submitochrondrial Particles*, CRC Press, Boca Raton, pp 1-35
Ruiz *et al.* 1997, *Bull. Environ. Contam. Toxicol. 59:* 619-625
Wang and Templeton, 1996, *Toxicol. Appl. Pharmacol. 139(1):* 115-121
Watanabe and Imai, 1981, *Anal. Chem. 55:* 1786-1791

## Claims

1. A method for detecting a toxicant comprising a heavy metal or heavy metal ion in a sample, said method comprising contacting said sample putatively containing said toxicant with a nucleic acid molecule bound to a fluorescent dye and screening for either dissociation of binding between said nucleic acid molecule and said dye or inhibition of binding between said nucleic acid molecule and said dye wherein said dissociation or inhibition of binding is indicative of the presence of said toxicant.

2. A method according to claim 1, wherein said dye is selected from the group consisting of acridine orange and ethidium bromide.

3. A method according to claim 1 or 2, wherein said nucleic acid molecule is immobilized to a solid support comprising glass, polystyrene, polymethacrylate, cellulose, nylon, polyvinylchloride or polypropylene.

4. A method according to claim 3 wherein the solid support is polystyrene or polymethacrylate.

## Patentansprüche

1. Verfahren zur Detektion eines Giftstoffs umfassend ein Schwermetall oder ein Schwermetallion in einer Probe, wobei das Verfahren umfasst,
Inkontaktbringen der Probe, welche mutmaßlich den Giftstoff enthält mit einem an einen Fluoreszenzfarbstoff gebundenem Nukleinsäuremolekül und
Durchmustern hinsichtlich entweder Dissoziation der Bindung zwischen dem Nukleinsäuremolekül und dem Farbstoff oder Hemmung der Bindung zwischen dem Nukleinsäuremolekül und dem Farbstoff,
wobei Dissoziation oder Hemmung der Bindung für das Vorliegen des Giftstoffs indikativ ist.

2. Verfahren gemäß Anpruch 1, wobei der Farbstoff ausgewählt ist, aus der Gruppe, welche aus Acridinorange und Ethidiumbromid besteht.

3. Verfahren nach Anspruch 1 oder 2, wobei das Nukleinsäuremolekül auf einem festen Träger immobilisiert ist, welcher Glas, Polystyrol, Polymethacrylat, Zellulose, Nylon, Polyvinylchlorid oder Polypropylen umfasst.

4. Verfahren nach Anpruch 3, wobei der feste Träger Polystyrol oder Polymethacrylat ist.

## Revendications

1. Méthode de détection d'une substance toxique comprenant un métal lourd ou un ion de métal lourd dans un échantillon, ladite méthode comprenant la mise en contact dudit échantillon contenant de manière putative ladite substance toxique avec une molécule d'acide nucléique liée à une sonde fluorescente et le criblage pour soit la dissociation de la liaison entre ladite molécule d'acide nucléique et ladite sonde, soit l'inhibition de la liaison entre ladite molécule d'acide nucléique et ladite sonde, ladite dissociation ou inhibition de la liaison étant indicative de la présence de ladite substance toxique.

2. Méthode selon la revendication 1, ladite sonde étant choisie dans le groupe consistant en l'acridine orange et le bromure d'éthidium.

3. Méthode selon la revendication 1 ou 2, ladite molécule d'acide nucléique étant immobilisée sur un support solide comprenant le verre, polystyrène, polyméthacrylate, cellulose, nylon, polychlorure de vinyle ou polypropylène.

4. Méthode selon la revendication 3, le support solide étant le polystyrène ou le polyméthacrylate.
